# EUROPEAN PATENT APPLICATION

(11) **EP 2 957 225 A1**
(43) Date of publication of application: **23.12.2015**
(21) Application number: 14002103.1
(22) Date of filing: 18.06.2014
(51) Int. Cl.: A61B 5/022, A61B 5/00, G01L 5/10, G01L 1/22

(54) **Strain gauge device and equipment with such strain gauge devices**

(71) Applicant: STBL Medical Research AG, 8807 Freienbach (CH); Empa Swiss Federal Laboratories for Materials Science and Technology, 8600 Dübendorf (CH)
(72) Inventor: Hirt, Etienne, CH-8005 Zurich (CH); Clemens, Frank Jörg, CH-8600 Frauenfeld (CH); Melnykowycz, Mark, CH-8406 Winterthur (CH)
(74) Representative: Schaad, Balass, Menzl & Partner AG

(57) **Abstract**

The strain gauge device (20) comprises an elastic band (22), the strain of which is to be measured. The sensor (26) comprises at least one elongated measuring strand (38) changing resistivity in dependence of the strain of the band (22). The measuring strand (38) is mounted with pre-tension to the band (22) by means of a layer of glue disposed between the sensor (26) and the band (22). The equipment (10) for continually measuring the blood pressure of a user comprises at least one strain gauge device (20).

## Description

The present invention relates to a strain gauge device according to claim 1 and an equipment for continually measuring the blood pressure of a user for monitoring purposes according to claim 20.

A strain sensor to measure large strain in textiles is disclosed in the article "Sensor for Measuring Strain in Textile" by Corinne Mattmann, Frank Clemens and Gerhard Troester in Sensors 2008, 8, 3719-3732. The strain sensor consists of a mixture of 50wt-% thermal plastic elastomer (TPE) and 50wt-% carbon black particles and is fiber-shaped with a diameter of 0,315 mm. This strain sensor was designed to measure elongations in textiles. For the integration or attachment of the sensor thread to the textile, the sensor thread was temporarily attached to the textile by using adhesive tape. Thereafter the textile was attached to a cardboard with fixing pins under light tension in order to prevent shifting when using conductive glue and to prevent the building of ripples when applying silicone. The sensor was then connected to conductive threads using conductive epoxy. After a drying period the sensor was attached to the textile by means of silicone by using a palette-knife. After a curing period, the adhesive tape and the fixing pins were removed and the textile strain sensor was finished.

In this arrangement the fiber-shaped strain sensor is covered by silicone and latterly attached to the textile by means of the silicone. It inheres some risk of crack nucleation while under stress.

WO 2010/017973 A1 discloses an equipment and a method for continually measuring the blood pressure of a user for monitoring purposes. The equipment has at least one pressure sensor disposed for being placed onto the surface to the body of the user and being held thereto by means of a band. The attachment force is selected such that the pressure signal from the pressure sensor contains variations caused by the pulse. A band tension sensor generates an electrical signal depending on the attachment pressure. A microprocessor determines the diastolic and systolic blood pressure values form the pressure signal taking into account the signal from the band tension sensor.

It is an object of the present invention to provide a strain gauge device that reliably works within large strain ranges. It is a further object to provide an equipment with such a strain gauge device.

These objects are achieved by a strain gauge device according to claim 1 and an equipment according to claim 20.

The strain gauge device according to the present invention comprises a substrate having reversible flexibility at least in a measuring zone. The strain of the substrate in the measuring zone is to be measured while stretching of the substrate by means of tensile force acting on the substrate in a tensioning direction.

Thus, the tension in the substrate can also be evaluated be means of the strain measurement.

Preferably the substrate is designed as an elastic band or a stretch-band.

At least in the measuring zone the substrate and the elastic band, respectively, has a reversible flexibility (i.e. an elasticity) preferably between 100/130 and 100/300, more preferred within 100/150 and 100/250, particularly between 100/200 and 100/240. In this connection the denominator indicates the original length and the nominator denominates the final length of maximal elastic deformation.

The strain gauge device further comprises a sensor having at least one elongated measuring strand changing resistivity in dependence of the applied strain. The measuring strand is arranged at least approximately parallel to the substrate and the band, respectively, in the measuring zone and with its lengthwise direction at least approximately in the tensioning direction of the substrate and the band, respectively.

Preferably, the sensor comprises or is made of a thermoplastic elastomer (TPE) and carbon black particles.

Preferably, the sensor is mounted to the substrate and the band, respectively, by means of a layer of adhesive, in particular of glue. The adhesive and glue, respectively, is disposed between the sensor and the substrate and the band, respectively.

Further, the sensor is attached to the substrate such that the measuring strand is under an amount of pre-tension at least approximately in the tensioning direction, when the substrate is unstressed.

The pre-tension ensures that the measuring strand is always tensioned.

Preferably, the amount of pre-tension of the measuring strand is between 5% and 30%, more preferably between 7% and 20%, most preferably at least approximately 10%.

An amount of pre-tension of x% means that the length of the mounted or attached measuring strand is 100%+x% of the length of the unstressed measuring strand.

Preferably, the material of the sensor comprises, more preferably is a mixture of a thermoplastic elastomer (TPE) and carbon black particles in the amount of 10wt-% - 60wt-%, preferably of 40wt-% - 55wt-%. It is more preferred an amount of at least approximately 50wt-% thermoplastic elastomer (TPE) to 50wt-% carbon black particles. The sensor is preferably formed in one piece of this material.

A mixing ratio of 50wt-% to 50wt-% leads to a monotonically increasing curve of the electrical resistivity of the sensor in dependence of the strain and to an adequate extensibility.

The material of the sensor can comprise, preferably can be a mixture of a thermoplastic elastomer (TPE) and electrical conductive particles, preferably particles of a metal or other inorganic material; preferred are particles of copper, silver, indium tin oxide or fluorine tin oxide, in the amount of 10wt-% - 60wt-%, preferably 40wt-% - 55wt-%, more preferably at least approximately 50wt-%. Preferably, the sensor is formed in one piece of this material.

Preferably, the sensor has a thickness equal to or less than 0.7 mm in order to minimize the risk of crack. More preferred the thickness is between 0.1 and 0.5 mm, most preferred of at least approximately 0.3 mm.

The whole sensor has preferably the same thickness.

Preferably, the measuring strand of the sensor has a width between 1 mm and 2 mm and a length between 7 mm and 20 mm, preferably 8 mm and 15 mm, especially of at least approximately 10 mm.

In top view, the form of the measuring strand is preferably rectangular.

The width of the measuring strand can be smaller than 1 mm; however, the width is preferably equal to or greater than 0.1 mm. Further, the length of the measuring strand can be smaller or greater than the above mentioned values; i.e. the length can be between 1 mm and 30 mm.

The above mentioned dimensions of the sensor and the measuring strand, respectively, and the composition of the material lead to a simple handling as well as to a good crack resistance and convenient electrical resistivity.

In a preferred embodiment the sensor comprises at least two parallel arranged measuring strands electrically connected in series, preferably particularly two measuring stands connected at one end by means of a common connecting strand so that the sensor has the shape of an U. The embodiment with just two measuring stands offers very simple possibilities for electrical connections.

The thickness and width of the connecting strand are preferably the same as of the measuring strand.

The sensor has a reversible flexibility of preferably at least 100/200 (i.e. 100%), more preferably of at least 100/250 (i.e. 150%).

The sensor and the measuring strand, respectively, preferably has a reversible flexibility similar to that of the substrate and the band, respectively.

In a preferred embodiment the sensor is area-wide attached to the substrate and the band, respectively, by the layer of adhesive, in particular of glue.

A relatively stiff attachment can be achieved by using an instant adhesive, for example Super Glue (alkylcyanacrylate). Nevertheless, a more elastic attachment can be advantageous. In this case, the use of rubber, thermoplastic elastomer (TPE) or polyurethane (PU) as glue is preferred. Silicone and synthetic rubber such as Plasti Dip^{®} is also suitable. The elasticity can further be tuned by the choice of the thickness of the layer of the glue.

Preferably, the measuring strand has, as seen in the tensioning direction, two end regions and these two end regions are connected to the substrate by the layer of adhesive or glue. Suitable are the same materials as mentioned in the previous paragraph.

The end regions have preferably at least approximately the same length as the width of the measuring strand.

In a preferred embodiment the attachment by the layer of adhesive or glue only takes place in the end regions.

In a preferred embodiment a gap, for example an air-gap, is limited by the layer of adhesive or glue in the end regions, the substrate and the band, respectively, and the measuring strand. Thus, the sensor and the measuring strand, respectively, is free of a connection with the substrate and the band, respectively, between the attached end regions.

Preferably, a space limited by the layer of adhesive or glue in the end regions, the substrate and the band, respectively, and the measuring strand is filled with a bonding layer, preferably liquid rubber, connecting the measuring strand with the substrate.

Preferably, this bonding layer elastically connects the substrate and the measuring strand, respectively, and the substrate and the band, respectively, between the attached end regions.

For this elastic connection, as bonding layer a synthetic rubber (for example Plasti Dip^{®}, Rubber Dip^{®}, Plasti Dip^{®} Electric Tape), nature rubber, silicone, thermoplastic elastomer (TPE), polyurethane (PU) or resin (e.g. epoxy) can be used.

The elastic connection can further be tuned by the choice of the thickness of the bonding layer.

In case the bonding layer should provide a more stiff connection or attachment an instant adhesive, for example Super Glue (alkylcyanacrylate) is also suitable.

Preferably, for the layer of glue and the bonding layer different materials are used in a strain gauge device.

Preferably, two reinforcement layers are disposed on the substrate with a distance there between, as seen in the tensioning direction, and the end regions are attached to the reinforcement layer by the layer of adhesive or glue. The reinforcement layer creates a material area of a higher stiffness with respect to the residual sections of the substrate and the band, respectively, and the sensor in order to shield the material area from excessive deformation.

This embodiment also reinforces electrode areas in which the sensor and the measuring strand, respectively, is connected to wires or electrical conductor paths for the electrical connection with an electronic circuit. The electronic circuit is designed for the processing of the signal generated by the sensor.

For example, the electronic circuit comprises a microprocessor in the equipment for continually measuring the blood pressure of a user for monitoring purposes.

Stiffness increase in the sense of a reinforcement layer may be due to using a thicker layer of the substrate or the band, respectively, as compared in the residual regions.

Another possibility for creating the reinforcement layer is the use of a (thin) layer of higher stiffness material. For this purpose for example particle reinforced polymer composite, synthetic rubber (for example Plasti Dip^{®}, Rubber Dip^{®}, Plasti Dip^{®} Electric Tape), nature rubber, silicone, thermoplastic elastomer (TPE), polyurethane (PU) or instant adhesive such as Super Glue (alkylcyanacrylate) are suitable.

Preferably, a space limited by the reinforcement layers, the layer of adhesive or glue in the end regions, the substrate and the measuring strand is filled with a bonding layer, preferably liquid rubber, connecting the measuring strand with the substrate.

Preferably, the bonding layer elastically connects the substrate and the measuring strand, respectively, and the substrate and the band, respectively, between the attached end regions and reinforcement layers.

For this elastic connection, as the bonding layer a synthetic rubber (for example Plasti Dip^{®}, Rubber Dip^{®}, Plasti Dip^{®} Electric Tape), nature rubber, silicone, thermoplastic elastomer (TPE), polyurethane (PU) or resin (e.g. epoxy) can be used.

In case the bonding layer should provide a more stiff connection or attachment an instant adhesive, for example Super Glue (alkylcyanacrylate) is also suitable.

Preferably, the layer of adhesive or glue, the bonding layer and the reinforcement layer of a strain gauge device are made of different materials.

In general, the bonding layer has the lowest stiffness of the adjacent materials while the reinforcement layer has the highest.

Preferably, the substrate has a stiffness approximately equal to or greater than the stiffness of the sensor material.

The bonding layer preferably has a stiffness at least equal to or lower than the sensor material.

The reinforcement layer preferably has a stiffness significantly greater than the other materials in the arrangement.

Preferably, the reinforcement layer has a stiffness greater than the combined stiffness of the adjacent materials, and therefore, greater than the combination of the sensor, bonding layer, and substrate as arranged stacked together. Therefore, ideally the reinforcement layer has at a minimum, a stiffness 2-5 times greater than the individual substrate, sensor, or bonding layer material, but it could be higher, at least 10-20 times greater.

The difference in stiffness between the individual layers may be adjusted by modifying the materials used in those layers (for example, by using a material with a higher Young's Modulus than the adjacent materials), but also by modifying the thickness of each individual layer.

The stiffness of the different layers can be adjusted by using different material combinations, for example, by the use of hard and soft rubber, thermoplastic elastomer or silicone. Additionally, the stiffness of the entire arrangement can be adjusted by modifying the thickness of the individual layers.

The reinforcement layer will generally have a smaller thickness than, or a thickness at least equal to the thickness of the sensor or substrate (substrate layer) in the arrangement. The substrate will generally have the largest thickness or a thickness at least equal to the thickness of each individual layer. In a preferred arrangement, the bonding layer will have a thickness smaller than either the sensor or the substrate, and additionally will have the lowest stiffness, or be at least equal to the stiffness of the sensor (sensor layer). If the material used for the reinforcement layer has a similar stiffness to the material stiffness of the individual bonding layer and sensor, then it would need to have a layer thickness of at least 2-6 times greater than thickness of that individual layer and sensor in order to have the larger required stiffness.

In a preferred embodiment the reinforcement layer extends at least approximately across the whole width of the substrate and the elastic band, respectively. By this, a good force transmission can be reached.

Preferably, the reinforcement layer has a width, measured in the tensioning direction, between 3 mm and 8 mm, more preferred of at least approximately 5 mm.

Preferably, the measuring strand - preferably the whole sensor - when attaching to the substrate and the band, respectively, is plastically deformed by a pre-straining process. This can preferably be achieved by pre-straining the sensor and the measuring strand, respectively, in the measuring direction between 50% and 200%, preferably between 80% and 150%, especially at least approximately 100%. An amount of pre-straining of x% means that the sensor and the measuring strand, respectively, is strained to a length of 100%+x% of the original, unstressed length.

After the pre-straining the sensor and the measuring strand, respectively, is released so that the recovered sensor and the measuring strand, respectively, can afterwards be attached to the substrate and the band, respectively, with pre-tension regarding the recovered length after pre-staining.

It is also possible to pre-strain the flat-belt (i.e. the bulk material) at the same amount as mentioned above before punching out the sensors.

In a preferred embodiment the substrate and the band, respectively, has flexibility following the measuring zone and / or the sensor on both sides, as seen in the tensioning direction, for at least approximately 10 mm, preferably for at least 15 mm to 20 mm.

Thus, at a larger distance to the sensor and the measuring strand, respectively, as mentioned above, the substrate and the band, respectively, can have a lesser flexibility, viz. a higher stiffness, without a negative influence on the sensor and its accuracy and reliability.

For measuring the electrical resistivity of the sensor and thus the change of the resistivity of the sensor in dependence of the strain, the sensor is preferably connected with a current supply or a current source so that the sensor is flown through by a predefined electrical current. In this case, the voltage between the two ends of the sensor is proportional to the resistivity. Thus, a simple measurement of this voltage delivers a proportion of the strain.

A preferred equipment for continually measuring the blood pressure of a user for monitoring purposes comprises at least one pressure sensor suitable for resting against a site on the external surface of the body of the user, continuously measuring the pressure at the site influenced by the blood pressure and generating a corresponding electrical contact-pressure signal and a strain gauge device according to the present invention. The substrate is an elastic band - or at least a portion therefrom - suitable for encompassing the body and holding the pressure sensor against the surface at the site with safe, functional contact. The strain gauge device is suitable for continuously measuring the strain, and thus the tensile stress in the band and generating a corresponding electrical band-tension signal. The equipment further comprises an electronic circuit with a current supply, a microprocessor for establishing a diastolic and a systolic blood-pressure value based on the contact pressure signal and taking into account the band-tension signal, and an output device for displaying or outputting the blood-pressure values.

The equipment is preferably designed as disclosed in document WO 2010/017973 A1, the disclosure of which is herewith incorporated by reference, whereby the strain gauge device corresponds to the present invention.

Preferably, the electronic circuit energizes the strain gauge device, i.e. the sensor with the predetermined current and measures the voltage as described above.

An equipment for continually monitoring a patient regarding reflux comprises at least one strain gauge device according to the present invention. The substrate, preferably an elastic band is suitable for encompassing the throat of the patient. During monitoring the elastic band is in functional contact with the throat. The strain gauge device is suitable for continuously measuring the strain of the band and generating a corresponding electrical band-tension signal. The equipment further comprises the electronic circuit with a current supply, a microprocessor for establishing a reflux value from the band-tension signal and an output device for displaying or outputting the reflux value.

Preferably, the electronic circuit energizes the strain gauge device, i.e. the sensor with the predetermined current and measures the voltage as described above.

An equipment for continually monitoring a patient's respiratory status, in particular regarding apnoea, comprises at least one strain gauge device according to the present invention. The substrate, preferably an elastic band, i.e. a chest strap, is suitable for encompassing the chest of the patient. During monitoring the elastic band is in functional contact with the chest. The strain gauge device is suitable for continuously measuring the strain of the band and generating a corresponding electrical band-tension signal. The equipment further comprises the electronic circuit with a current supply, a microprocessor for establishing a respiratory value from the band-tension signal and an output device for displaying or outputting the respiratory status.

Preferably, the electronic circuit energizes the strain gauge device, i.e. the sensor with the predetermined current and measures the voltage as described above.

An equipment for continually monitoring muscle or joint movement of a user comprises at least one strain gauge device according to the present invention. The substrate is an elastic band suitable for encompassing the muscle and overlapping the joint, respectively. The strain gauge device is suitable for continuously measuring the strain of the band and generating a corresponding electrical band-tension signal. The electronic circuit comprises a current supply, a microprocessor for establishing a length value from the band-tension signal and an output device for displaying or outputting the length value.

Preferably, the electronic circuit energizes the strain gauge device, i.e. the sensor with the predetermined current and measures the voltage as described above.

An equipment for continually measuring the contact pressure of thrombosis stockings for monitoring purposes and prevention of pressure marks comprises a number of strain gauge devices according to the present invention. The substrate is a thrombosis stocking, the strain gauge devices are mounted on the stocking and are suitable for continuously measuring the strain of the thrombosis stocking and generating corresponding electrical tension signals. The equipment further comprises an electronic circuit with a current supply, a microprocessor for establishing contact pressure values from the tension signals and an output device for displaying or outputting the contact pressure values.

Preferably, the electronic circuit energizes the strain gauge devices, i.e. the sensors with the predetermined current and measures the voltage as described above.

The invention will be explained in more details on the basis of the embodiments illustrated in the drawing, in which, in a purely schematic fashion,
- figure 1: shows a top view of an equipment for continually measuring the blood pressure comprising two strain gauge devices for continually measuring the strain and thus the tensile stress in the band;
- figure 2: shows in a top view one of the strain gauge devices according to figure 1;
- figure 3: shows in lateral view a first possible structure of the strain gauge device;
- figure 4: shows in lateral view a second possible structure of the strain gauge device;
- figure 5: shows in lateral view a third possible structure of the strain gauge device;
- figure 6: shows in lateral view a fourth possible structure of the strain gauge device;
- figure 7: shows a top view of an equipment as a vital function data device comprising two strain gauge devices for continually measuring the strain and thus the tensile stress in the band;
- figure 8: shows in a front view a human body as well as equipment and vital function data devices, respectively, arranged at different sites; and
- figure 9: shows in a lateral view the human body as well as vital function data devices arranged at different sites.

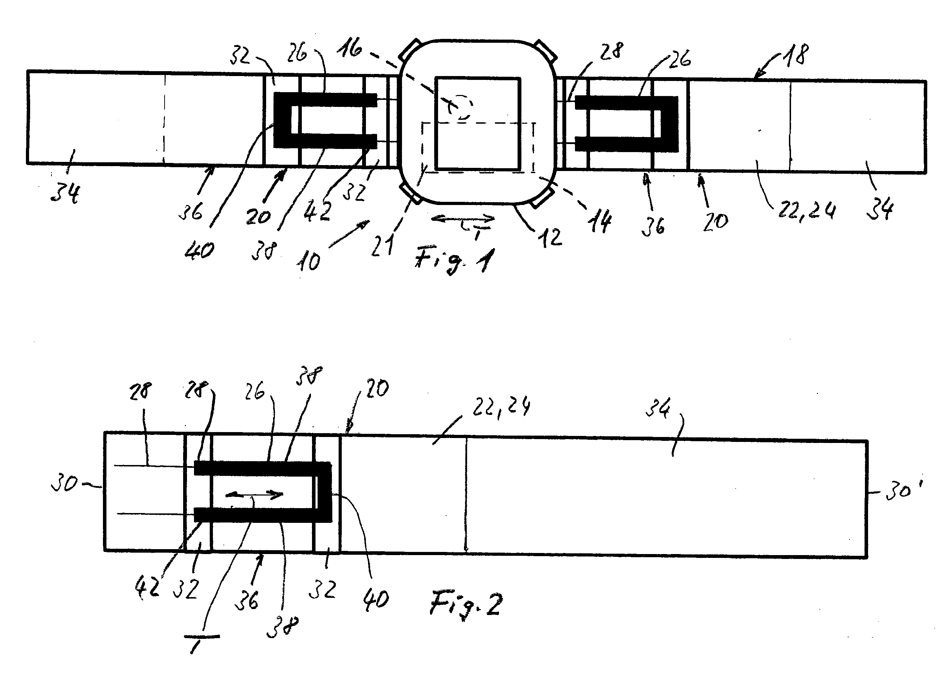

Figure 1 shows an equipment 10 for continually measuring the blood pressure of a user for monitoring purposes.

The equipment 10 comprises a housing 12 with a bearing plate 14 beyond which a pressure sensor 16 protrudes. The pressure sensor is suitable for resting against a site on the external surface of the body of the user, in the present case on the surface of an arm, for continuously measuring the pressure at the site influenced by the blood pressure and for generating a corresponding electrical pressure signal.

At the housing 12 the ends of two parts of a band 18 are fixed. The wristband 18 is suitable for encompassing the arm and holding the pressure sensor 16 against the surface at the site with safe, functional contact. Each of these two parts of the wristband 18 constitutes a strain gauge device 20 - a so called band-tension sensor - for continuously measuring the tensile stress in the wristband 18 and generating a corresponding electrical band-tension signal or band-strain signal.

An electronic circuit 21 with a current supply, a microprocessor for determination of a diastolic and systolic blood-pressure value from the pressure signal, taking into account the band tension signal/band strain signal, and an output device for displaying or outputting the blood-pressure values, is arranged in the housing 12.

Such an equipment 10 - with the exception of a different strain gauge device 20 - is disclosed in the document WO 2010/017973 A1 the disclosure of which is hereby introduced into the present disclosure by reference.

In the embodiment shown in figures 1 and 2, each strain gauge device 20 comprises an elastic band 22, defining a substrate 24, and a sensor 26 attached to the band 20 for measuring the strain and thus the mechanical tension in the band 22.

The sensor 26 is connected to the electronic circuit 21 by means of wires or electrical conductor paths 28.

The sensor 26 changes the electrical resistivity in dependence or the applied strain.

For measuring the electrical resistivity of the sensors 26 and thus the change of the resistivity of the sensors 26 in dependence of the strain, the sensors 26 are connected with a current supply or a current source of the electronic circuit 21 via the wires or electrical conductor paths 28 so that the sensors 26 are flown through by a predefined electrical current. A voltage measuring portion of the electronic circuit 21 continually measures the voltage between the wires or electrical conductor paths 28, respectively, and inputs the voltage-values to the microprocessor. In this case, the voltage between the two ends of the sensors 26, i.e. the wires or electrical conductor paths 28 is proportional to the resistivity. Thus, a simple measurement of this voltage delivers a proportion of the strain.

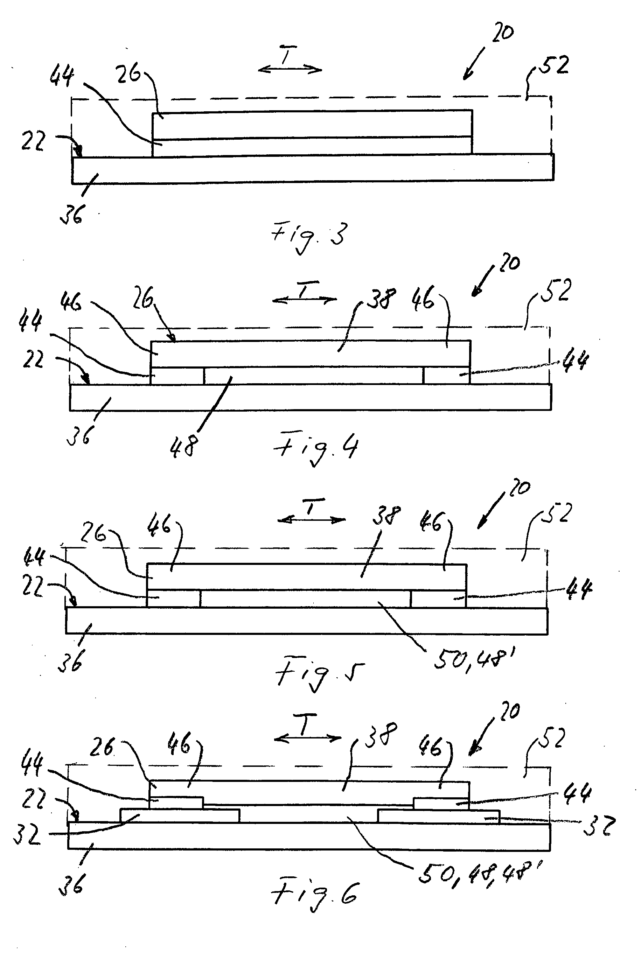

There are different possibilities for the attachment of the sensor 26 on the band 22 as shown in figures 3 to 6. Figures 1 and 2 show the same possibility as also represented in figure 6.

Three different elastic bands 22 were tested and evaluated as being suitable for the strain gauge device 20, namely White Elastic 31070/25, White Elastic 31062/23 and Black Elastic 29930/25 produced by JHCO Elastic AG, CH-4800 Zofingen, having extensibilities of 100/230, 100/230 and 100/220, respectively. The width of the bands 22 is 25 mm and 23 mm, respectively, as mentioned in the product names.

In the embodiment shown in the figures 1, 2 and 6, at a distance of about 15 mm to 20 mm from the end 30 of the band 22 fastened to the housing 12, a linear reinforcement layer 32 is applied on the band 22. The enforcement layer 32 has a width of about 5 mm and runs across the whole width of the band in a direction at right angles to the longitudinal direction of the band that corresponds to a tensioning direction T.

As seen in the same direction, at a distance of about 10 mm as from the enforcement layer 32 a further enforcement layer 32 is applied to the band 22. The dimensions of both enforcement layers 32 are similar.

A Velcro^{®} strip 32 (hook - and loop fastener) is fixed to the band 22. The Velcro^{®} strip reaches from the other end 30' of the band up to distance of about 15 mm to the further reinforcement layer 30. Thus, in the region of the Velcro^{®} strip the band 22 is practically inelastic and the band 22 constitutes between the Velcro^{®} strip 34 and first end 30 a measuring zone 36 having flexibility and elasticity.

The material of the sensor 26 is a mixture of thermoplastic elastomer (TPE) - for example SEBS-Block copolymer THERMOLAST K^{®} (FD-Series), Compound No. TF 7 - ATL produced by KRAIBURG TPE GmbH, Germany, and carbon black powder - for example ENSACO 250 produced by TIMCAL, Belgium. The density of the TPE was 0.89 g/cm2 and of the carbon black powder 1.8±0.2 g/cm2. The primary particle size and the specific surface area of the carbon black powder were 54 nm and 65±5 cm2/g, respectively.

After melting the thermoplastic part of TPE, carbon black powder was added and subsequently homogenized and dispensed into the polymer during 1 hour of 180° C.

After compounding, a flat-belt was produced by using an extension die having a rectangular orifice with a clearance of 0.3 mm. Because of the swelling, the thickness of the flat-belt increased to about 0.315 mm.

For obtaining on the one hand a monotonically increasing resistance curve in dependence of the strain and on the other hand a sensor material having a similar extensibility as the band 22 a mixture of 50wt-% TPE and 50wt-% carbon black particles was used.

From the flat-belt sensors 26 were punched out.

The sensor 26 has two parallel measuring strands 38 of a length of 14 mm and a width of 10 mm. The distance between the measuring strands is about 3 mm. The two measuring strands 38 are connected at one end to one another by means of a common connecting strand 40. Thus the sensor 26 has the shape of an U, as seen in top view, whereby the free end regions of the measuring strands 38 form electrode areas 42 for the electrical connection with the wires and electrical conductor paths 28, respectively.

Thus, a resistance of approximately 156 to 205 Ω/cm of the U-shaped sensor 26 in resting state was achieved.

This kind of sensor 26 should have a thickness of less than 0.7 mm. The thickness is preferably 0.1 to 0.5 mm most preferably about 0.3 mm.

The width of the measuring strands 38 is preferably 1 mm to 2 mm.

A free expandable section of the measuring strands 38 in the resting state is in the shown embodiment about 10 mm, but can be selected shorter or longer, however preferably between 5 mm and 20 mm.

However, a sensor 26 with only one measuring strand 38 or more than two measuring strands 38 is also possible.

The sensor 26 is attached to the band 22 by means of a layer of glue 44 as shown in the figures 3 to 6 disposed between the sensor 26 and the band 22 and with an amount of pre-tension in the longitudinal direction of the band 22 and thus in the tensioning direction T.

In the embodiments shown in the figures the amount of pre-tension is 10%. What this means is that, when attached to the band 22, in the resting state of the band 22, the length of the measuring strands 38 is 110% of the length in its (unloaded) resting state.

However, the pre-tension can be chosen differently between the preferred limits mentioned in the introductory portion and the claims.

When attaching the sensor 26 to the band 22, the sensor 26 or the bulk material of the sensor has undergone plastic deformation during a pre-straining process. That can be achieved by pre-straining the bulk material or the sensor 26, in particular the measuring strands 38 in the tensioning direction T.

It is possible to pre-strain either the sensor 26 and its measuring strands 38 or the extruded and cooled down flat-belt before the punching out of the sensors 26.

It is also possible to produce the sensor 26 by injection molding, casting, compression molding or 3D printing.

The measuring strands 38 have, as seen in the tensioning direction T, two end regions 46. At least these end regions 46 of the sensor 26 are attached to the band 22. Preferably, the end regions 26 have a length similar to the width of the measuring strands 38.

In the embodiment comprising a U-shaped sensor 26 and shown in the figures, at least the free end regions 46 coinciding with the electrode areas 42, the connecting strand 40 and the near side end regions 46 are attached to the band 22 by means of the layer of glue 44.

Figure 3 shows a portion of the measuring zone 36 of the band 22, the sensor 26 and the layer of glue 44 there between. The sensor 26 is and, thus, the measuring strands 38 are area-wide attached to the band 22 by this layer of glue 26.

If an instant adhesive, for example, Super Glue, is used a rather stiff, inflexible attachment of the sensor 26 is produced.

However, also the use of an elastic layer of glue 44, for example synthetic rubber (Plasti Dip^{®} Spray), silicone, polyurethane (PU), thermoplastic elastomer (TPE) or rubber, leads to reliable measuring results and additionally has the advantage of reducing stress in the sensors 26.

Figure 4 shows the same portion of the band 22 as exhibited in figure 3, the sensor 36 and the layer of glue 44. However, only the end regions 46 of the measuring strands 38 and the connecting strand 40 (see figures 1 and 2) are attached to the band 22 by means of the layer of glue 44.

For the layer of glue 44 the same materials can be used as mentioned above in connection with the description of figure 3.

Between at one hand the measuring strands 38 of the sensor 26 and the band 22 and on the other hand the layer of glue 44 in the end regions 46 a gap is existent.

Figure 5 shows the same structure of the strain gauge device 20 as figure 4 with the exception that the space 48' corresponding to the gap 48 is filled with a bonding layer 50. Thus, there exists a connection between the measuring strands 38 and band 22 over the whole length of the measuring stand 38 but with different elasticity due to the use of different materials for the layer of glue 44 and the bonding layer 50.

For the bonding layer 50 the same materials can be used as mentioned above in connection with the layer of glue 44; preferably a liquid rubber is used.

The structure of the strain gauge device 20 shown in figure 6 corresponds to that of figures 1 and 2.

The two reinforcement layers 32 are applied to the band 22 in the measuring zone 36 as already disclosed in connection with the description of figures 1 and 2.

The layer of glue 44 for attaching the sensor 26 is applied to the reinforcement layer 32 on the side facing away from the band 22. As can be seen, the width of the layers of glue 44 is preferably smaller than the width of the reinforcement layer 32, as measured in the tensioning direction T.

The reinforcement layers 32 have preferably a higher stiffness with respect to the rest of the band 32 and the sensor material in order to shield the end regions 46 of the measuring strands 38 and thus the electrode areas 42 from excessive deformation when stretching the band.

In the region of the reinforcement layer 32 the elasticity of the band 22 is hence reduced whereas in the residual regions of the measuring zone 36, in particular between the reinforcement layers 32, the elasticity is not influenced.

For the reinforcement layer 32 liquid silicone rubber can be used.

However, also different materials such as synthetic rubber (Plasti Dip^{®}), rubber, silicone, polyurethane (PU), thermoplastic elastomer (TPE), Super Glue (instant adhesive) and particle reinforced polymer composite can be used as reinforcement layer 32.

Preferably, the reinforcement layer 32 and the layer of glue 44 consist of different materials.

The stiffness of the layer of glue 44 is preferably smaller than the stiffness of the reinforcement layer 32.

The gap 48 delimited by the band 22, the sensor 26, the reinforcement layers 31 and the layers of glue 44 can be free of material. Thus, the sensor 26 is exclusively attached to the band 22 via the layer of glue 44 and the reinforcement layers 32.

However, the space 48' corresponding to the above mentioned gap 48 can be filled with a bonding layer 50 as described in connection with figure 5.

It is noted that the reinforcement layer 32 can have a form different to a linear one.

It is also possible to cover the strain gauge device 20, in particular the sensor 26 by means of a layer of elastomer, for example Plasti Dip^{®}, or an elastic coating film; see the elastic cover 52 illustrated by broken lines in the figures 3 to 6.

In the equipment 10 as shown in figure 1, the electrical band-tension signals / band-strain signals produced by the sensors 26 of the two strain gauge devices 20 are preferably independently analyzed and compared by the microprocessor in order to enhance the reliability and thus then accuracy of the blood pressure values.

However, the equipment 10 can be equipped with only one strain gauge device 20.

In all embodiments, the elongated measuring strands 38 are arranged parallel to the band 22 and its longitudinal direction corresponds to the tensioning direction T of the band 22.

The stiffness of the band 22 can be tuned by adding polymer spray layers. It is also possible to tune only the regions of the band 22 between the reinforcement layers 32 and the Velcro^{®} or only the region of the band 22 between the reinforcement layers 32. The same holds true regarding the layer of glue 44 in case of the absence of the reinforcement layers 32.

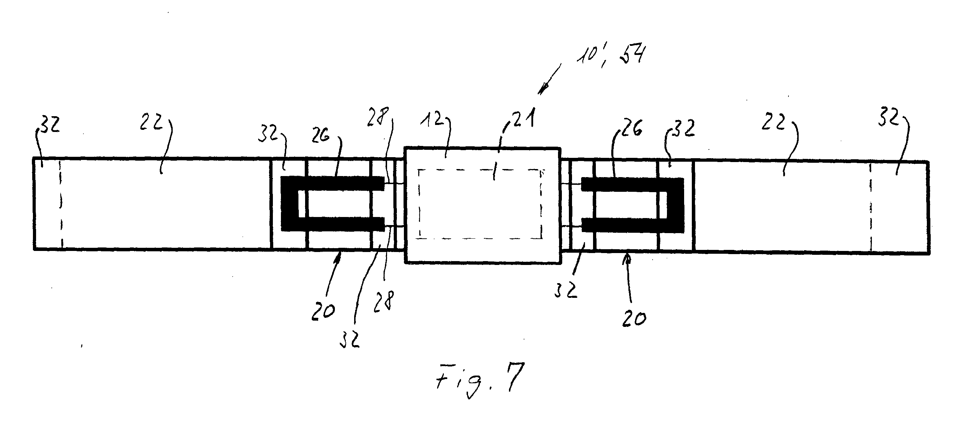

The equipment 10' shown in figure 7 - a vital function data device 54 - comprises a housing 12. At the housing 12 two parts of an elastic band 22 are fixed. The elastic band 22 is suitable for encompassing the external surface of the body of the user at a desired site and with tensile stress 10 so that the vital function data device 54 is safely held at the site influenced by the respective function of the body of the user to be monitored.

Each of the two parts of the band 22 constitutes a strain gauge device 20 - a so called band-tension sensor - for continuously measuring tensile stress in the band 22 and generating a corresponding electrical band-tension signal or band-strain signal.

The electronic circuit 21 with the current supply, the micro- processor for determination of desired values from the band-tension/band-strain signal and the output device for displaying or outputting the values, is arranged in the housing 12. The strain gauge devices 22 are designed as the strain gauge devices 22 shown in figures 1 to 6 and described above.

The sensors 26 of the strain gauge devices are connected to the electronic circuit 21 by means of wires or electrical conductor paths 28.

For measuring the electrical resistivity of the sensors 26 and thus the change of the resistivity of the sensor 26 in dependence of the strain, the sensors 26 are connected with a the current supply so that the sensors 26 are flown through by a defined electrical current. The electronic circuit 21 comprises means for measuring the voltage between the two wires and electrical conductor paths 28, respectively, and thus the voltage across the sensors 26.

This voltage is led to the microprocessor and is proportional to the resistivity of the sensors 26 and thus the change of the voltage is also a proportion to the change of the resistivity due to the change of the strain. Thus, a simple measurement of this voltage delivers a proportion of the strain.

As already described in connection with the figures 1, 2 and 6, in the embodiment shown in figure 7 at a distance of about 15 mm to 20 mm from the end of the band 22 fastened to the housing 12, linear reinforcement layers 32 are applied on the band 22. The reinforcement layers 32 have a width of about 5 mm and run across the whole width of the band in a direction of right angles to the longitudinal direction of the band 22 corresponding to the tensioning direction T.

As seen in the same direction, at a distance of about 10 mm as from the reinforcement layers 32 further reinforcement layers 32 are applied to the band 22. The dimensions of the four reinforcement layers 32 are similar. A Velcro^{®} strip (hook - and loop fastener) is fixed to the band 22 in the free end portions of the band 22. The distance between the further reinforcement layer 32 and the Velcro^{®} strip 32 is at least about 15 mm. For at last in the measuring zones 36 the band 32 has flexibility and elasticity.

In lieu of Velcro^{®} strips 32 other known fasteners for coupling the two end regions of the band 22 can be used.

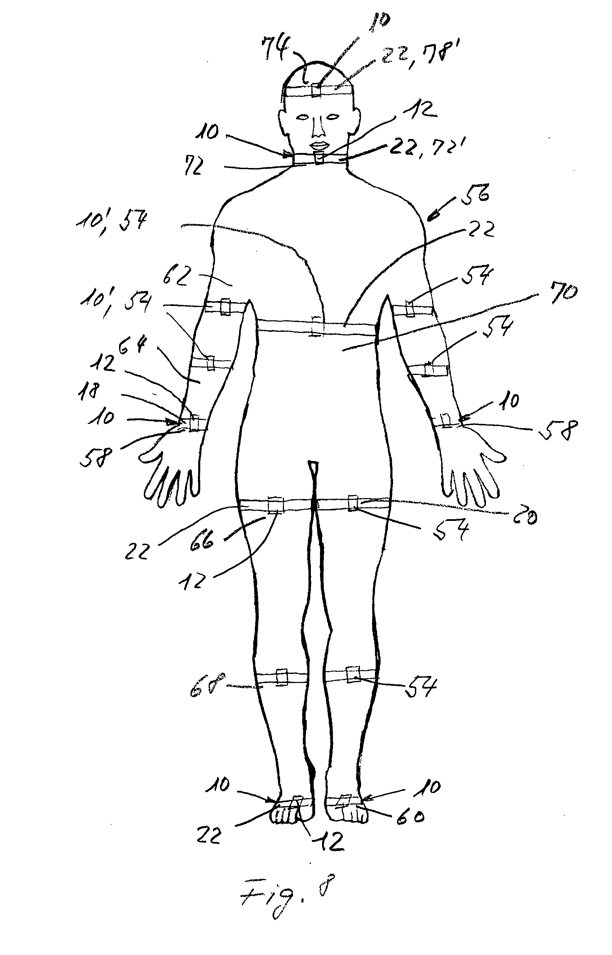

Figure 8 shows - in a front view - the human body 56 of the user of equipment 10 and equipment 10' / vital function data device 54.

At both wrists 58 an equipment 10 for continually measuring the blood pressure is shown. The housings 12 are held at the site on the external surface of the body by wristbands 18. The design and function of the equipment 10 is the same as shown in figures 1 to 6 and described above.

In figure 8 two further such equipment 10 are shown at the feet near the roots of the toes 60. By continually measuring the blood pressure the blood flow is monitored. The housings 12 are held at the sites by means of the elastic bands 22.

Vital function data devices 54 -i.e. equipment 10' - are worn at the upper arms 62, at the upper end region of the forearms 64, at the thighs 66 and at the calf of the lower legs 68.

Elastic bands 22 hold the devices 54 with the housings 12 at the sites. The move of the respective muscles induces changes in the circumference of the body 56 at the respective site. That leads to changes of the tensile strength of the bands 22 what is continuously monitored by means of the strain gauge devices 20 and the electronic circuit 21. The electrical band-tension signals of the strain gauge devices are led to the microprocessor of the electronic circuit 21 that establishes length values from that signals. These length values are outputted or displayed by the output device of the electronic circuit.

An equipment 10' in the form of a vital function device 54 as shown in figure 7 is used for continually monitoring the respiratory status of the user. In particular the existence or not existence of apnoea can be determined by a continually long-term measurement of the expansion and contraction of the thorax.

The vital function device 54 is held at the site by means of a chest strap designed as an elastic band 22. The preferred site is at the largest circumference of the chest 70.

The expansion and contraction of the thorax induces changes of the tensile strength of the chest strap what is continually monitored by means of the strain gauge devices 20 and the electronic circuit 21.

The electrical band-tension signals of the strain gauge devices are led to the microprocessor that establishes respiratory values from the band-tension signals. These respiratory values are displayed or outputted by means of the output device of the electronic circuit 21.

A further equipment 10' according to figure 7 is arranged at the neck 72 of the user's body 56. This equipment 10' for continually monitoring a patient regarding reflux comprises at least one strain gauge device 20 according to the present invention. The substrate 24, preferably an elastic band 22 is suitable for encompassing the throat of the patient. During monitoring the elastic band 22 is in functional contact with the throat. The strain gauge device 20 - there are preferably two strain gauge devices 20 - is suitable for continuously measuring the strain of the band 22, i.e. the substrate 24, and generating a corresponding electrical band-tension signal. The equipment 10' further comprises the electronic circuit 21 with a current supply, a microprocessor for establishing a reflux value from the band-tension signal and an output device for displaying or outputting the reflux value.

The vital function data device 54 can also be used for continually monitoring the movement of joints of the user. In this case the vital function data device 54 extends on the external surfaces of the body of the user to the respective joint and the ends of the elastic hands 22 are fixed to the body on both sides of the respective joint at a distance to the joint. That fixation can for example be achieved by fixation bands 76. Adhesive tapes can also be used.

The movement of the joints leads to a change of the length of the elastic bands 22 and thus to a change of the tensile strength. The strain gauge devices 20 are suitable for continuously measuring the strain of the bands 22 and generating a corresponding electrical band-tension signal.

The microprocessor of the electronic circuit 21 establishes length values from these band-tension signals. By means of the output device of the electronic circuit 21 the length values are displayed or outputted.

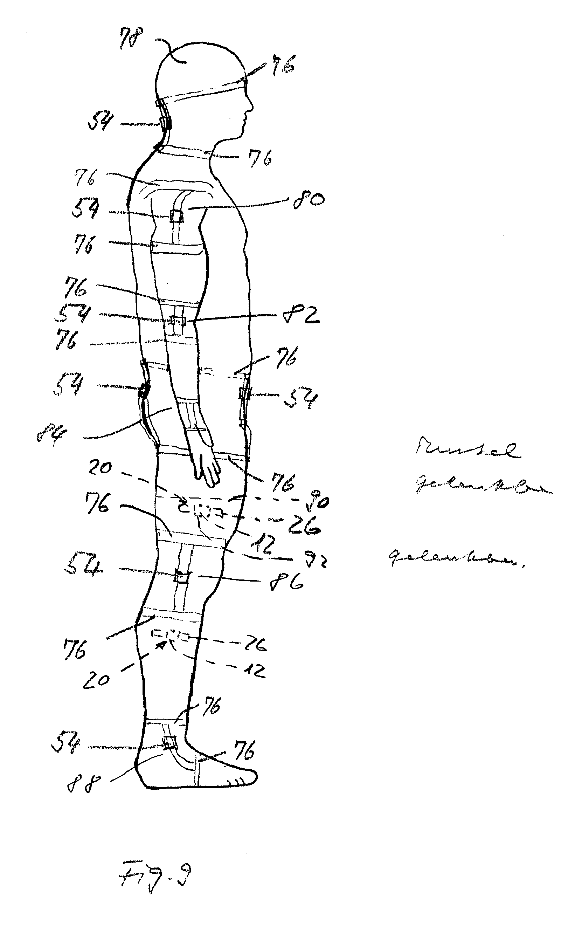

Figure 9 shows vital function data devices 54 / equipment 10' for continually monitoring the movement of the head 78, the shoulder joint 80, the elbow 82, the hip joint 84 - on the stomach side and the back side -, the knee joint 86 and the ankle joint 88.

In figure 9 reference numeral 90 indicates thrombosis stockings. The elastic material itself of the stockings 90 can serve as the substrate 24 of the strain gauge device 20. In this case, the sensors 26 are applied to the stockings 90 with pre-tension as described above and shown in figures 3 to 7. Preferably two sensors 26 are arranged in each case at two opposite sides of the respective housing 12 in the same manner as described above and shown in Figure 7; see strain gauge devices 20 indicated in broken lines in figure 9.

The strain gauge devices 20 connected with the microprocessor of the electronic circuit 21 in the respective housing 12 are suitable for continuously measuring the strain of the thrombosis stocking at different sides of the user's legs and generating corresponding electrical sensor signals. The electronic circuit 21 comprises the current supply, the microprocessor for establishing pressure values from the tension signals, and the output device for displaying or outputting the pressure values

Preferably, the vital function data device comprises a thermometer or a temperature sensor 92 commented with the microprocessor in order to preferably continually measure and monitor the temperature of the user's body at the respective side.

The contact pressure of the thrombosis stockings 90 and pressure sores as well as galls can be detected.

Thus, the present invention is also related to the following objects:
A) An equipment for continually monitoring a user's respiratory status, in particular regarding apnoea, comprising a strain gauge device 20 according the present invention (as defined in the claims 1 to 19), wherein the substrate is an elastic band 22 - a chest strap - suitable for encompassing the chest 70 of the user and being in safe, functional contact with the chest 70; the strain gauge device 20 is suitable for continuously measuring the strain of the band 22 and generating a corresponding electrical band-tension signal; and an electronic circuit 21 with a current supply, a microprocessor for establishing a respiratory value from the band-tension signal, and an output device for displaying or outputting the respiratory status.
B) An equipment for continually monitoring muscle or joint movement of a user, comprising at least one strain gauge device 20 according the present invention (as defined in the claims 1 to 19), wherein the substrate 24 is an elastic band 22 suitable for encompassing the muscle and overlapping the joint, respectively, the strain gauge device 20 is suitable for continuously measuring the strain of the band 22 and generating a corresponding electrical band-tension signal; and an electronic circuit 21 with a current supply, a microprocessor for establishing a length value from the band-tension signal, and an output device for displaying or outputting the length value.
C) An equipment for continually measuring the contact pressure of thrombosis stockings for monitoring purposes and prevention of pressure marks, comprising a number of strain gauge devices 20 according to the present invention (as defined in the claims 1 to 19), wherein the substrate 24 is a thrombosis stocking, the strain gauge devices 20 are mounted on the stocking and are suitable for continuously measuring the strain of the thrombosis stocking 90 and generating corresponding electrical tension signals; and an electronic circuit 21 with a current supply, a microprocessor for establishing contact pressure values from the tension signals, and an output device for displaying or outputting the contact pressure values.

## Claims

1. Strain gauge device comprising a substrate (24) having flexibility at least in a measuring zone (36), the strain of the substrate (24) is to be measured while stretching in a tensioning direction (T), and a sensor (26) comprising at least one elongated measuring strand (38) changing resistivity in dependence of the strain and arranged at least approximately parallel to the substrate (24) in the measuring zone (36) and aligned at least approximately in the tension direction (T), wherein the measuring strand (38) is mounted to the substrate (24) with an amount of pre-tension at least approximately in the tensioning direction (T).

2. Strain gauge device according to claim 2, wherein the amount of pre-tension is between 5% and 70%, preferably between 7% and 20%, most preferably at least approximately 10%.

3. Strain gauge device according to claim 1 or 2, wherein the material of the sensor (26) comprises, preferably is a mixture of a thermoplastic elastomer (TPE) and carbon black particles in the amount of 10wt-% - 60wt-%, preferably of 40wt-% - 55wt-%, more preferably at least approximately 50wt-%, and the sensor (26) is formed in one piece of this material.

4. Strain gauge device according to claim 1 or 2, wherein the material of the sensor (26) comprises, preferably is a mixture of a thermoplastic elastomer (TPE) and electrical conductive particles, preferably particles of a metal or other inorganic material, preferably copper, silver, indium tin oxide or fluorine tin oxide, in the amount of 10wt-% - 60wt-%, preferably 40wt-% - 55wt-%, more preferably at least approximately 50wt-%, and the sensor (26) is formed in one piece of this material.

5. Strain gauge device according to one of the claims 1 to 4, wherein the sensor (26) has a thickness equal to or less than 0.7 mm, preferably between 0.1 and 0.5 mm, most preferably of at least approximately 0.3 mm.

6. Strain gauge device according to one of the claims 1 to 5, wherein the measuring strand (38) of the sensor (26) has a width between 1 mm and 2 mm and a length between 7 mm and 20 mm, preferably 8 mm and 15 mm, especially of at least approximately 10 mm.

7. Strain gauge device according to one of the claims 1 to 6, wherein the sensor (26) comprises at least two parallel arranged measuring strands (38) electrically connected in series, preferably particularly two measuring stands (28) connected at one end by means of a common connecting strand (40) so that the sensor (26) has the shape of an U.

8. Strain gauge device according to one of the claims 1 to 7, wherein the sensor (26) has a reversible flexibility of at least 100/150, preferably of at least 100/200, in particular of at least 100/250.

9. Strain gauge device according to one of the claims 1 to 8, wherein the measuring strand (38) is mounted to the substrate (24) by means of a layer of adhesive, in particular glue (44) disposed between the sensor (26) and the substrate (24).

10. Strain gauge device according to claim 9, wherein the sensor (26) is area-wide attached to the substrate (24) by the layer of adhesive or glue (44), preferably by an instant adhesive or synthetic rubber, silicone, polyurethane (PU), thermoplastic elastomer (TPE) or rubber.

11. Strain gauge device according to claim 9 or 10, wherein the measuring strand (38) has, as seen in the tensioning direction (T), two end regions (46) and these two end regions (46) are connected to the substrate by the layer of adhesive or glue (44), preferably by an instant adhesive or synthetic rubber, silicone, polyurethane (PU), thermoplastic elastomer (TPE) or rubber.

12. Strain gauge device according to claim 11, wherein a gap (48) is limited by the layer of adhesive or glue (44) in the end regions (46), the substrate (24) and the measuring strand (38).

13. Strain gauge device according to claim 11, wherein a space (48') limited by the layer of adhesive or glue (44) in the end regions (46), the substrate (24) and the measuring strand (38) is filled with a bonding layer (50), preferably a liquid rubber, connecting the measuring strand (38) with the substrate (24).

14. Strain gauge device according to claim 11, wherein two reinforcement layers (32) are disposed on the substrate (24) with a distance there between, as seen in the tensioning direction (T), and the end regions (46) are attached to the reinforcement layer (32) by the layer of adhesive or glue (44).

15. Strain gauge device according to claim 14, wherein a space (48') limited by the reinforcement layers (32), the layer of adhesive or glue (44) in the end regions (46), the substrate (24) and the measuring strand (38) is filled with a bonding layer (50), preferably a liquid rubber, connecting the measuring strand (38) with the substrate (24).

16. Strain gauge device according to one of the claims 1 to 15, wherein the substrate (24) is an elastic band (22), preferably a stretch-band.

17. Strain gauge device according to claims 14 and 16 or 15 and 16, wherein the reinforcement layer (32) extends at least approximately across the whole width of the elastic band (22).

18. Strain gauge device according one of the claims 1 to 17, wherein the measuring strand (38), preferably the whole sensor (26) is plastically deformed by a pre-straining process, preferably by pre-straining in the measuring direction (T) between 50% and 200%, preferably between 80% and 150%, more preferably at least approximately 100%.

19. Strain gauge device according to one of the claims 1 to 16, wherein the substrate (24) and the band (22), respectively, has flexibility following the sensor (26) on both sides, as seen in the tensioning direction (T), for at least approximately 10 mm, preferably for at least 15 mm to 20 mm.

20. Equipment for continually measuring the blood pressure of a user for monitoring purposes, with at least one pressure sensor (16) suitable for resting against a site on the external surface of the body of the user, continuously measuring the pressure at the site influenced by the blood pressure and generating a corresponding electrical pressure signal; and a strain gauge device (20) according to one of the claims 1 to 19 , wherein the substrate (24), preferably an elastic band (22) is suitable for encompassing the body and holding the pressure sensor (16) against the surface at the site with safe, functional contact; and the strain gauge device (20) is suitable for continuously measuring the strain of the substrate (24; 22) and generating a corresponding electrical band-tension signal; and an electronic circuit (21) with a current supply, a microprocessor for establishing a diastolic and a systolic blood-pressure value from the contact-pressure signal taking into account the band-tension signal, and an output device for displaying or outputting the blood-pressure values.
